# EUROPEAN PATENT APPLICATION

(11) **EP 4 552 572 A1**
(43) Date of publication of application: **14.05.2025**
(21) Application number: 23208167.9
(22) Date of filing: 07.11.2023
(51) Int. Cl.: A61B 5/243, A61B 5/00

(54) **PROVIDING A FETAL CARDIAC SIGNAL**

(71) Applicant: Koninklijke Philips N.V., 5656 AG Eindhoven (NL)
(72) Inventor: LEUSSLER, Christoph Günther, 5656AG Eindhoven (NL); HAHN, Artur, Eindhoven (NL)
(74) Representative: Philips Intellectual Property & Standards

(57) **Abstract**

A monitoring system (100) for providing a fetal cardiac signal includes a plurality of magnetic field sensors (110_{1..i}), and one or more processors (120). The one or more processors (120) are configured to receive composite magnetocardiogram, MCG, signals generated by the magnetic field sensors (110_{1..i}). The composite MCG signals comprise a maternal MCG signal representing magnetic fields generated by a beating heart of a pregnant mother and a fetal MCG signal (130) representing magnetic fields generated by a beating heart of her fetus. The one or more processors (120) are also configured to analyse the composite MCG signals to extract the fetal MCG signal (130) from the composite MCG signals; and to output the fetal MCG signal (130) to provide the fetal cardiac signal.

## Description

### FIELD OF THE INVENTION

The present disclosure relates to providing a fetal cardiac signal. A monitoring system, a medical imaging system, a computer-implemented method, and a computer program product, are disclosed.

### BACKGROUND OF THE INVENTION

The functioning of the heart is often investigated by means of an electrocardiogram, or "ECG". An ECG is a type of cardiac signal that is obtained by placing electrodes on the skin of a subject. The electrodes detect electrical signals that are generated as a result of cardiac muscle depolarization and re-polarization during the cardiac cycle. Other types of sensors are also used to obtain cardiac signals in order to investigate the functioning of the heart, including ultrasound sensors.

However, there remain challenges to obtaining reliable cardiac signals using existing sensors, in particular when obtaining cardiac signals of a fetus of a pregnant mother. Many of these challenges originate from the weak nature of the signals that are generated by a fetal heart. In the case of an ECG, the ability to obtain reliable cardiac signals is also hampered by the poor sensitivity of the electrodes. The electrodes also require contact with the pregnant mother, and these can be cumbersome to attach. Wires are typically used to couple the ECG signals from the electrodes to a monitoring system, and these wires can interfere with clothing. During medical imaging procedures, the wires can also obstruct anatomical features and degrade image quality. Similar challenges are encountered when ultrasound sensors are used to obtain cardiac signals. Cardiac signals that are obtained using ultrasound sensors may also require a different interpretation to those obtained using electrodes because they provide a measurement of motion rather than a direct measurement of the heart's muscular activity.

Consequently, there remains a need to improve the detection of cardiac signals from a fetus.

### SUMMARY OF THE INVENTION

According to one aspect of the present disclosure, a monitoring system for providing a fetal cardiac signal, is provided. The monitoring system includes:
- a plurality of magnetic field sensors; and
- one or more processors;
- wherein the one or more processors are configured to:
   receive composite magnetocardiogram, MCG, signals generated by the magnetic field sensors, the composite MCG signals comprising a maternal MCG signal representing magnetic fields generated by a beating heart of a pregnant mother and a fetal MCG signal representing magnetic fields generated by a beating heart of her fetus;
   analyse the composite MCG signals to extract the fetal MCG signal from the composite MCG signals; and
- output the fetal MCG signal to provide the fetal cardiac signal.

In the above monitoring system, magnetic field sensors are used to detect magnetic fields generated by a beating heart of a pregnant mother, and magnetic fields generated by a beating heart of her fetus. The magnetic fields are detected in the form of composite magnetocardiogram, MCG, signals. The inventors have determined that some of the drawbacks of electrode-based sensors can be mitigated by using magnetic field sensors. For instance, magnetic field sensors may provide improved sensitivity to the electrical activity of a heart over electrode-based sensors. As compared to ultrasound-based sensors, magnetic field sensors may provide a more accurate representation of the muscular activity of the heart because magnetic field sensors do not rely on reflected ultrasound signals, and which can be contaminated by other movements within the body such as a organ movement, and movement of the fetus. Thus, the use of magnetic field sensors may contribute to providing a more accurate fetal cardiac signal over ultrasound-based sensors.

The inventors have determined that measurements of fetal MCG signals are typically detected in the form of a composite MCG signal that includes contributions from both a maternal MCG signal and a fetal MCG signal. This is due to the relatively close proximity of the two hearts. In such a composite MCG signal, the maternal MCG signal can even be relatively larger than the fetal MCG signal. This is due to the relatively larger size of the heart of the pregnant mother as compared to the heart of the fetus. In the above monitoring system, a fetal MCG signal is extracted from the composite MCG signals. This results in the provision of a more reliable fetal MCG signal, and therefore a more reliable cardiac signal.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a monitoring system 100 for providing a fetal MCG signal 130, in accordance with some aspects of the present disclosure.
Fig. 2 is a flowchart illustrating an example of a computer-implemented method of providing a fetal MCG signal, in accordance with some aspects of the present disclosure.
Fig. 3 is a schematic diagram illustrating an example of a medical imaging system 200 and a monitoring system 100 for providing a fetal MCG signal 130, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the systems disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there remains a need to improve the detection of cardiac signals from a fetus.

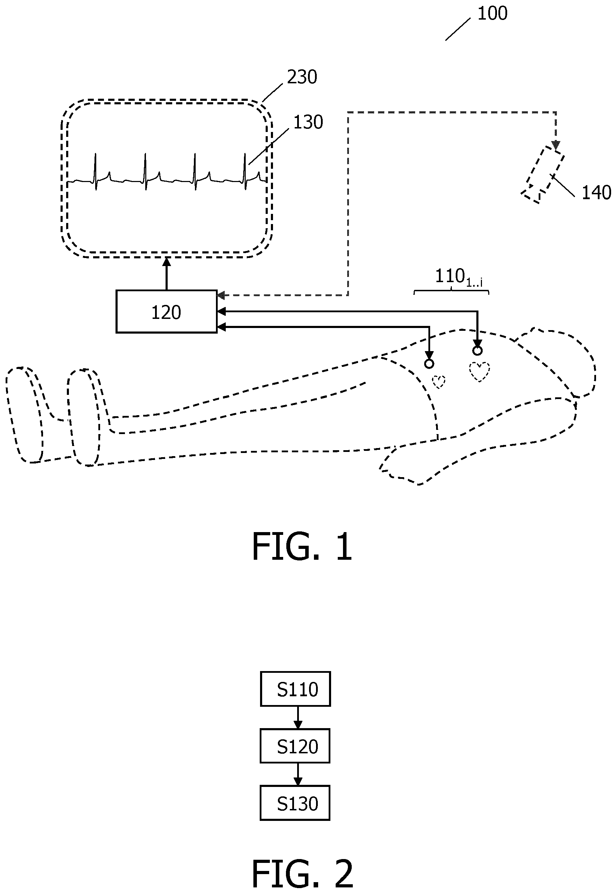

Fig. 1 is a schematic diagram illustrating an example of a monitoring system 100 for providing a fetal MCG signal 130, in accordance with some aspects of the present disclosure. Fig. 2 is a flowchart illustrating an example of a computer-implemented method of providing a fetal MCG signal, in accordance with some aspects of the present disclosure. It is noted that operations described as being performed by the one or more processors 120 of the monitoring system 100 illustrated in Fig. 1, may also be performed in the method illustrated in Fig. 2. Likewise, operations described in relation to the method described with reference to Fig. 2 may also be performed by the one or more processors 120 of the monitoring system 100 illustrated in Fig. 1. With reference to Fig. 1, and Fig. 2, the monitoring system 100 for providing a fetal cardiac signal, comprises:
- a plurality of magnetic field sensors 110_{1..i}; and
- one or more processors 120;
- wherein the one or more processors 120 are configured to:
   receive S110 composite magnetocardiogram, MCG, signals generated by the magnetic field sensors 110_{1..i}, the composite MCG signals comprising a maternal MCG signal representing magnetic fields generated by a beating heart of a pregnant mother and a fetal MCG signal 130 representing magnetic fields generated by a beating heart of her fetus;
   analyse S120 the composite MCG signals to extract the fetal MCG signal 130 from the composite MCG signals; and
   output S130 the fetal MCG signal 130 to provide the fetal cardiac signal.

In the above monitoring system 100, magnetic field sensors are used to detect magnetic fields generated by a beating heart of a pregnant mother, and magnetic fields generated by a beating heart of her fetus. The magnetic fields are detected in the form of composite magnetocardiogram, MCG, signals. The inventors have determined that some of the drawbacks of electrode-based sensors can be mitigated by using magnetic field sensors. For instance, magnetic field sensors may provide improved sensitivity to the electrical activity of a heart over electrode-based sensors. As compared to ultrasound-based sensors, magnetic field sensors may provide a more accurate representation of the muscular activity of the heart because magnetic field sensors do not rely on reflected ultrasound signals, and which can be contaminated by other movements within the body such as a organ movement, and movement of the fetus. Thus, the use of magnetic field sensors may contribute to providing a more accurate fetal cardiac signal over ultrasound-based sensors.

The inventors have determined that measurements of fetal MCG signals are typically detected in the form of a composite MCG signal that includes contributions from both a maternal MCG signal and a fetal MCG signal. This is due to the relatively close proximity of the two hearts. In such a composite MCG signal, the maternal MCG signal can even be relatively larger than the fetal MCG signal. This is due to the relatively larger size of the heart of the pregnant mother as compared to the heart of the fetus. In the above monitoring system, a fetal MCG signal is extracted from the composite MCG signals. This results in the provision of a more reliable fetal MCG signal, and therefore a more reliable cardiac signal.

The monitoring system 100 is described in more detail below.

The monitoring system 100 includes a plurality of magnetic field sensors 110_{1..i}. The magnetic field sensors generate MCG signals. MCG signals are similar to the ECG signals that are generated by electrodes because, as with ECG signals, MCG signals provide a direct measurement of the heart's muscular activity. The use of magnetic field sensors also avoids some of the motion-induced artifacts that may contaminate ultrasound-based measurements of cardiac signals. Thus, the magnetic field sensors provide reliable cardiac signals. Moreover, unlike the use of electrode-based sensors, or ultrasound sensors, the use of magnetic field sensors provides the opportunity to perform contact-less measurements of cardiac signals, as described in the examples below.

Various types of magnetic field sensors are contemplated for use as the magnetic field sensors 110_{1..i} in the monitoring system 100. For example, inductive sensors, or quantum sensors such as optically pumped magnetometer, OPM, sensors, or nitrogen vacancy diamond sensors, may be used.

The inductive sensors may be provided in various forms, including in the form of a coiled electrical path. The coiled electrical path may be coupled to an amplifier to amplify electrical signals that are generated in the coiled electrical path a result of detected magnetic fields.

As mentioned above, quantum sensors may also be used as the magnetic field sensors 110_{1..i} in the monitoring system 100. As compared to inductive sensors, quantum sensors may be used to provide increased sensitivity to magnetic fields. Quantum sensors typically include particles in a balanced state that is affected by small variations in magnetic fields. The particles can take the form of neutral atoms, trapped ions, and solid-state spins, for example. Examples of such quantum magnetic field sensors are described in a document by Meyer, D. H., et al., "Waveguide-coupled Rydberg spectrum analyzer from 0 to 20 GHz", Phys. Rev. Applied 15: 014053; 2021; and in a document by Aslam, N., et al., "Quantum sensors for biomedical applications", Nature Reviews Physics 5: 157-169; 2023; and in a document by Chen X-D., at al., "Quantum enhanced radio detection and ranging with solid spins", Nature Communications, 2023, https://doi.org/10.1038/s41467-023-36929-8. The thin film devices that are reported in the document by Aslam, N., et al., cited above, have a sensitivity of 3 pico Tesla/^Hz. This level of sensitivity facilitates the detection of magnetic fields that are generated by cardiac muscle.

In general, quantum sensors may convert detected magnetic fields into electrical signals, or into optical signals. The electrical, or optical, signals may then be amplified. In the former case, a bridge circuit and/or an amplifier circuit may be used to amplify the electrical signals. An example of a quantum magnetic sensor in which detected magnetic fields are converted into electrical signals is marketed under the name "Nivio xMR sensor" by the TDK Corporation, Chuo-ku, Tokyo, Japan.

An example of a quantum sensor in which the detected magnetic fields are converted into optical signals is an optically pumped magnetometer, OPM, sensor. An example of an OPM is described in the document by Coussens, T. et al. "Modular optically-pumped magnetometer system", 2021, arXiv:2106.05877v1. An OPM sensor typically includes an optical (e.g. glass) chamber comprising an Alkali metal gas such as Rubidium. The chamber may have dimensions in the order of millimetres. In operation, Rydberg states are prepared, i.e. "pumped", and probed, using two lasers, e.g. blue, and near-infrared. The absorption of laser irradiation within the chamber changes with local electromagnetic fields in accordance with Rydberg state superposition. One of the two lasers is used to pump the OPM, and the other laser is used to probe the OPM and to thereby measure the magnetic field. Magnetic fields are measured by using an optical detector to measure optical properties of the probe beam after it has probed the OPM. Both lasers may be located remotely from the glass chamber in which the magnetic fields are detected. For instance, optical fibers may be used to couple the lasers, and also the associated optical detector, to the optical chamber. The ability to remotely couple the lasers and the associated optical detector to the OPM, combined with the lack of metal in an OPM, makes the OPM well-suited for use in imaging situations such as MRI, CT, and X-ray imaging procedures.

An alternative type of quantum sensor to the above-mentioned OPM is a nitrogen vacancy "NV" diamond sensor An example of an NV diamond sensor is marketed by Quantum Diamonds, Munich. Nitrogen vacancy diamond sensors typically include a (synthetic) diamond crystal comprising an arrangement of nitrogen vacancy centers. The diamond crystal may have dimensions in the order of millimetres. In an NV diamond sensor, Rydberg state are typically prepared with green laser. NV diamond sensors are part of the class of "solid-state spin" sensors using electronic spin defects in semiconductors.

In one example, the magnetic field sensors 110_{1..i} in the monitoring system 100 comprise optically pumped magnetometer, OPM, sensors, or nitrogen vacancy diamond sensors, and the monitoring system comprises one or more lasers configured to irradiate the OPM sensors, or the nitrogen vacancy diamond sensors, for generating the composite MCG signals. In this example, the one or more processors 120 are configured to generate control signals for controlling the irradiation of the OPM sensors, or the nitrogen vacancy diamond sensors, by the one or more lasers.

In this example, there may be one pump laser and one probe laser for each OPM sensor in the monitoring system 100. Alternatively, the monitoring system 100 may include a single pump laser and/or a single probe laser. In the latter case, the control signals may cause the single pump laser and/or the single probe laser to pump and/or probe multiple OPM sensors in the monitoring system 100 sequentially. Using a single pump laser and/or a single probe laser facilitates the provision of a more compact monitoring system 100.

The use of various configurations of the magnetic field sensors 110_{1..i} in the monitoring system 100, is contemplated. In one configuration, the plurality of magnetic field sensors 110_{1..i} comprises a first magnetic field sensor for coupling to a thorax of the pregnant mother, and a second magnetic field sensor for coupling to an abdomen of the pregnant mother. Providing sensors for coupling to these locations helps to distinguish the fetal MCG signal 130 from the maternal MCG signal. An example of this configuration is illustrated in the monitoring system 100 illustrated in Fig. 1. In this configuration, the sensors may each include an interface that is shaped for coupling to the respective part of the body. In this configuration, the magnetic field sensors 110_{1..i} may include an adhesive material for attaching the magnetic field sensors 110_{1..i} to the body.

As mentioned above, an advantage of using magnetic field sensors over e.g. electrode-based sensors, and also ultrasound sensors, is that magnetic field sensors do not require contact with the body. In one configuration, it is proposed to mount the magnetic field sensors 110_{1..i} to a support structure. The support structure may support the magnetic field sensors such that the sensors are held in positions that are mutually fixed, and also remote from the body. In one example, the support structure is provided by a portion of a medical imaging system. For instance, the magnetic field sensors may be mounted to a panel that defines a cover, or a bore of a medical imaging system. Alternatively, the support structure may comprise an attachment for coupling the support structure to a patient support, such as a patient table. In another example, the support structure may comprise a robotic arm.

In some examples, techniques are proposed for reducing interference to the composite MCG signals arising from background magnetic fields. These techniques help to improve the sensitivity of the monitoring system 100 to MCG signals, and/or improve its specificity to MCG signals. The background magnetic fields may originate from sources such as the earth's magnetic field, electronic equipment such as medical imaging systems, communication devices, and so forth. The background magnetic fields may be static, or they may be alternating. For instance, the earth's magnetic field, and also the Bo field from a magnetic resonance imaging, MRI, system give rise to static background magnetic fields. By contrast, the gradient coils of an MRI system, and communication devices give rise to alternating background magnetic fields.

In one example, the magnetic field sensors 110_{1..i} comprise magnetic shielding for reducing interference to the composite MCG signals from background magnetic fields.

Effective magnetic shielding materials typically have a high value of magnetic permeability. One example of such materials is MuMetal, which is a Nickel-Iron alloy. Some Nickel-Iron MuMetals that provide a high degree of magnetic shielding have more than 40%, of Nickel. The magnetic shielding may be arranged so as to shield the magnetic field sensors 110_{1..i} from background magnetic fields originating from directions other than the expected direction of the fetal heart-induced magnetic field. The magnetic shielding may attenuate both static and alternating background magnetic fields.

In another example, the monitoring system comprises a filter configured to filter the composite MCG signals for reducing interference to the composite MCG signals from background magnetic fields.

In this example, the filter may include a DC filter configured to attenuate signals arising from static background magnetic fields such as those described above. The filter may include a bandpass filter configured to attenuate alternating background magnetic fields at frequencies that are outside a range of frequencies that are expected for the composite MCG signals. For example, the filter may attenuate both DC signals arising from static background magnetic fields, and which occur at relatively lower frequencies than the range of frequencies that are expected for the composite MCG signals, and also alternating signals arising from alternating background magnetic fields such as those originating from electronic equipment, and which occur at relatively higher frequencies than the range of frequencies that are expected for the composite MCG signals.

In another example, the monitoring system includes one or more reference magnetic field sensors configured to detect background magnetic fields. In this example, the one or more processors 120 are configured to compensate for interference to the composite MCG signals from the background magnetic fields, using the detected background magnetic fields.

In this example, the one or more processors may compensate for interference by subtracting from the signals generated by the magnetic field sensors 110_{1..i} the signals generated by the reference magnetic field sensors in response to the background magnetic fields.

In another example, the magnetic field sensors 110_{1..i} each comprise at least one magnetic field compensation coil. In this example, the one or more processors 120 are configured to apply signals to the magnetic field compensation coils for reducing interference to the composite MCG signals from background magnetic fields.

In this example, the magnetic field compensation coil(s) may be provided by Helmholtz coils. For instance, the magnetic field sensors 110_{1..i} may each be surrounded by one or more pairs of Helmholtz coils. By way of an example, three pairs of Helmholtz coils may surround each magnetic field sensor, each pair being separated along one of the orthogonal axes x, y, and z. Compensation for magnetic field interference may be provided by adjusting the currents in each of the Helmholtz coils in response to measurements of the background magnetic fields. The currents in the Helmholtz coils generate magnetic fields within the volume encompassed by the coils, which has the effect of reducing, or nulling, the effect of the background magnetic fields on the corresponding magnetic field sensor. Active compensation for alternating background magnetic fields may be provided by adjusting the currents in real-time. Thus, the Helmholtz coils may be used to compensate for both static background magnetic fields, as well as alternating background magnetic fields.

In another configuration, interference to the magnetic field sensors 110_{1..i} from the magnetic fields generated by an MRI system is reduced by aligning the magnetic field sensors 110_{1..i} such that the MRI system's magnetic field direction (Bo typically being aligned with the scanner's longitudinal axis, often referred to as the z-direction) is perpendicular to the sensitive orientation of the sensor. Since the static and gradient magnetic field components generated by the MRI system are strongest along the z-direction, the proposed sensors may be oriented perpendicular to this axis, thereby avoiding the detection of strong magnetic flux through the sensors. During radiofrequency excitation (short RF pulses), the sensors may be switched off (in the case of optically controlled sensors) or de-tuned. Alternatively, filtering methods as those described above may be used reduce the amount of such high frequency contamination in the detected composite MCG signal.

A combination of the above techniques may also be used to reduce interference to the composite MCG signals arising from background magnetic fields.

Referring now to the operation S 110 mentioned above with reference to Fig. 1 and Fig. 2, in this operation the one or more processors 110 of the monitoring system 100 receive composite MCG signals. The composite MCG signals comprise a maternal MCG signal representing magnetic fields generated by a beating heart of a pregnant mother and a fetal MCG signal 130 representing magnetic fields generated by a beating heart of her fetus. As mentioned above, the inventors have determined that measurements of fetal MCG signals are typically detected in the form of a composite MCG signal that includes contributions from both a maternal MCG signal and a fetal MCG signal. This is due to the relatively close proximity of the two hearts. In such a composite MCG signal, the maternal MCG signal can even be relatively larger than the fetal MCG signal. This is due to the relatively larger size of the heart of the pregnant mother as compared to the heart of the fetus. In the above monitoring system, a fetal MCG signal is extracted from the composite MCG signals. This results in the provision of a more reliable fetal MCG signal, and therefore a more reliable cardiac signal.

The composite MCG signals may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber. For instance, in some examples the magnetic fields detected by the sensors are converted into electrical signals. In these examples, the composite MCG signals may be received by the one or more processors via an electrical cable. In other examples, the composite MCG signals may be converted into RF, or infrared signals, and the RF, or infrared, composite MCG signals received wirelessly by the one or more processors. In other examples it is contemplated to use optical signals to prepare, and also to probe, the magnetic field sensors. In these examples, the composite MCG signals may be received by the one or more processors via one or more optical fibers.

Referring now to the operation S 120 mentioned above with reference to Fig. 1 and Fig. 2, in this operation the one or more processors 110 of the monitoring system 100 analyse the composite MCG signals to extract the fetal MCG signal from the composite MCG signals.

Various techniques are contemplated for analysing the composite MCG signals in the operation S 120. For instance, in one example, one or more (bandpass) filters may be used to attenuate the maternal MCG signal from the composite MCG signals, and to thereby provide the fetal MCG signal. Fetal MCG signals tend to have a relatively higher fundamental frequency than maternal MCG signals, and so the (bandpass) filter may be used to attenuate the relatively lower frequency signals arising from alternating magnetic fields originating from the heart of the pregnant mother.

In another example, the composite MCG signals are analysed in the operation S 120 using a neural network.

In this example, the analysing S 120 the composite MCG signals comprises:
- inputting the composite MCG signals into a neural network; and
- generating the fetal MCG signal 130, from the composite MCG signals, in response to the inputting, using the neural network; and

- wherein the neural network is trained to generate the fetal MCG signal 130, from the composite MCG signals, using training data comprising a plurality of training composite signals comprising a maternal MCG signal representing magnetic fields generated by a beating heart of a pregnant mother and a fetal MCG signal 130 representing magnetic fields generated by a beating heart of her fetus, and for each training composite signal, a corresponding ground truth fetal MCG signal or a corresponding ground truth fetal ECG signal.

The neural network in this example may be provided by various architectures, including a recurrent neural network "RNN", encoder/ decoder architectures, variational autoencoders, and so forth. In general, the training of the neural network involves inputting the training data into the neural network, and iteratively adjusting the neural network's parameters until the trained neural network provides an accurate output. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem, including gradient descent, Quasi-Newton methods, and so forth. Various algorithms have been developed to implement these methods and their variants, including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers". These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

The training of a neural network is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

In the above example, the neural network may be trained to generate the fetal MCG signal 130 from the composite MCG signals, by:
- for each of a plurality of the training composite signals:
   inputting the training composite signal into the neural network;
   generating a fetal MCG signal 130, from the training composite signal, in response to the inputting, using the neural network; and
   adjusting parameters of the neural network based on a difference between the fetal MCG signal 130 generated by the neural network, and the corresponding ground truth fetal MCG signal, or the corresponding ground truth fetal ECG signal, respectively; and
- repeating the inputting, the generating, and the adjusting, until a stopping criterion is met.

The training data that is used to train the neural network may include traning composite signals and corresponding ground truth fetal MCG/ ECG signals from some tens, hundreds, thousands, or more, different subjects. The subjects may have a range of different ages, body mass index, co-morbidities, and so forth. The training composite signals and the corresponding ground truth fetal MCG/ ECG signals may have durations that are in the order of seconds, or minutes, or they may have a longer duration. The training composite signals and the corresponding ground truth fetal MCG/ ECG signals in the training data may be generated contemporaneously.

In one example, at least some of the training composite signals comprise synthetically-generated training composite signals, and the synthetically-generated training composite signals each represent a maternal MCG signal and a fetal MCG signal 130. In this example, the synthetically-generated training composite signals may be generated from real maternal MCG signals and corresponding real fetal MCG signals. Alternatively, the synthetically-generated training composite signals may be generated from real maternal ECG signals and corresponding real fetal ECG signals. The synthetically-generated training composite signals may be generated by summing, for example with random weights, the real maternal MCG/ ECG signal and the real fetal MCG/ ECG signal. The use of such synthetically-generated training composite signals may simplify the task of generating a large pool of training data.

In another example, the training data comprises a plurality of training composite signals comprising a maternal MCG signal representing magnetic fields generated by a beating heart of a pregnant mother and a fetal MCG signal representing magnetic fields generated by a beating heart of her fetus, and for each training composite signal, a corresponding ground truth fetal MCG. Providing the training data from a single type of sensor, i.e. using magnetic field sensors, may simplify the task of generating the training data.

In another example, the training data comprises a plurality of training composite signals comprising a maternal MCG signal representing magnetic fields generated by a beating heart of a pregnant mother and a fetal MCG signal representing magnetic fields generated by a beating heart of her fetus, and for each training composite signal, a corresponding ground truth fetal ECG. Thus, in this example, the neural network is trained to generate fetal MCG signals that resemble fetal ECG signals, i.e. cardiac signals that are generated using electrodes. Although MCG signals are similar to ECG signals, the fetal MCG signal that is generated by the trained neural network in this example may appear even more similar to a fetal ECG, which is the type of signal with which a physician is typically acquainted.

In another example, a vital signs signal(s) is inputted into the neural network and used, in combination with the composite MCG signals, to generate the fetal MCG signal 130. In this example, the monitoring system 100 includes at least one vital signs sensor 140, and the one or more processors 120 are configured to:
- receive at least one vital signs signal generated by the at least one vital signs sensor, the vital signs signal representing a vital sign of the pregnant mother;
- input the at least one vital signs signal into the neural network; and
- generate the fetal MCG signal 130, from the composite MCG signals, based further on the at least one vital signs signal; and
wherein the training data used to train the neural network further comprises for the at least one vital signs sensor, training vital signs signals corresponding to the training composite signals.

In this example, the vital signs signal(s) may represent one or more of: body temperature, pulse rate, respiration rate, and blood pressure. The vital signs sensor 140 may be provided by sensors such as a thermometer, an SPO2 sensor, a chest strap, a camera, a RADAR sensor, an accelerometer, a blood pressure monitor, and so forth. An example of a vital signs sensor in the form of a camera 140 that is used to extract body temperature, pulse rate, respiration rate, is illustrated in Fig. 1. The training vital signs signals may be generated contemporaneously with the training composite signals. At inference, the neural network may use correlations between the vital signs signal(s) and the composite MCG signals to provide an improved fetal MCG signal.

In another example, motion signal(s) are inputted into the neural network and used to generate the fetal MCG signal 130. In this example, the monitoring system 100 includes at least one motion sensor, and the one or more processors 120 are configured to:
- receive at least one motion signal generated by the at least one motion sensor, the motion signal representing a motion of the pregnant mother;
- input the motion signal into the neural network; and
generate the fetal MCG signal 130, from the composite MCG signals, based further on the at least one motion signal; and
- the training data used to train the neural network further comprises for the at least one motion sensor, training motion signals corresponding to the training composite signals.

In this example, the motion sensor may be coupled to the body of the pregnant mother. The motion sensor may be an accelerometer, or a gyroscope, for instance. The training motion signals may be generated contemporaneously with the training composite signals. At inference, the neural network may use correlations between the motion signal(s) and the composite MCG signals to provide an improved fetal MCG signal.

Referring now to the operation S130 mentioned above with reference to Fig. 1 and Fig. 2, in this operation the one or more processors 110 of the monitoring system 100 output S130 the fetal MCG signal 130. The fetal MCG signal 130 may be outputted in various ways, including to a display device such as to the monitor 230 illustrated in Fig. 1, or to a virtual/ augmented reality display device, or to a computer-readable storage medium, or to the internet, or to the Cloud, and so forth.

By providing the fetal MCG signal 130, the monitoring system 100 provides a cardiac signal that facilitates a physician to investigate the functioning of the fetal heart.
Various further examples are described below.

In one example, the operation of analysing S120 the composite MCG signals, also includes extracting the maternal MCG signal from the composite MCG signals. In this example, the one or more processors 120 are configured to output the maternal MCG signal to provide a maternal cardiac signal.

By providing the maternal MCG signal in accordance with this example, the monitoring system 100 facilitates a physician to investigate the functioning of the heart of the pregnant mother.

The maternal MCG signal may be extracted from the composite MCG signals in the analysing S 120 operation by performing operations corresponding to those described above for the maternal MCG signal instead of for the fetal MCG signal 130. Thus, in one example, one or more (bandpass) filters may be used to attenuate the fetal MCG signal from the composite MCG signals, and to thereby provide the maternal MCG signal. In another example, the composite MCG signals are analysed using a neural network in order to extract the maternal MCG signal from the composite MCG signals.

It is noted that in the examples described above, the monitoring system 100 may also include one or more of: a display, such as the monitor 230 illustrated in Fig. 1, for displaying the fetal MCG signal 130, other outputs generated by the one or more processors 120, and so forth; and a user input device (not illustrated in Fig. 1) configured to receive user input in relation to the operations performed by the one or more processors 120, such as a keyboard, a mouse, a touchscreen, and so forth.

In another example, a computer-implemented method of providing a cardiac signal, is provided. The method comprises:
- receiving S 110, from a plurality of magnetic field sensors 110_{1..i}, composite magnetocardiogram, MCG, signals generated by the magnetic field sensors 110_{1..i}, the composite MCG signals comprising a maternal MCG signal representing magnetic fields generated by a beating heart of a pregnant mother and a fetal MCG signal 130 representing magnetic fields generated by a beating heart of her fetus;
- analysing S 120 the composite MCG signals to extract the fetal MCG signal 130 from the composite MCG signals; and
   - outputting S 130 the fetal MCG signal 130 to provide the fetal cardiac signal.

In another example, a computer program product, is provided. The computer program product comprises instructions which when executed by one or more processors, cause the one or more processors to carry out a method of providing a cardiac signal. The method comprises:
- receiving S 110, from a plurality of magnetic field sensors 110_{1..i}, composite magnetocardiogram, MCG, signals generated by the magnetic field sensors 110_{1..i}, the composite MCG signals comprising a maternal MCG signal representing magnetic fields generated by a beating heart of a pregnant mother and a fetal MCG signal 130 representing magnetic fields generated by a beating heart of her fetus;
- analysing S 120 the composite MCG signals to extract the fetal MCG signal 130 from the composite MCG signals; and
- outputting S 130 the fetal MCG signal 130 to provide the fetal cardiac signal.

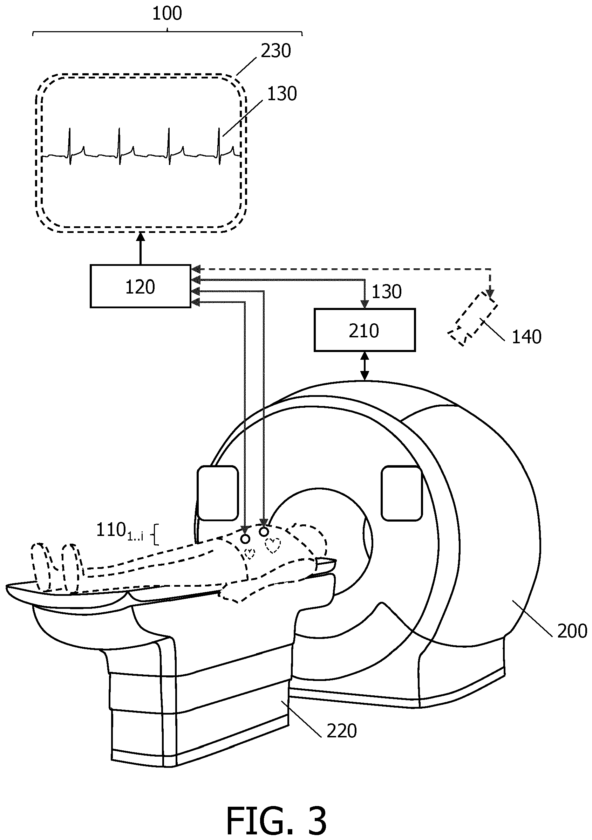

In another example, a medical imaging system that includes the monitoring system 100, is provided. The medical imaging system uses the fetal MCG signal 130 to trigger the acquisition of medical image data, or to select medical image data for reconstruction, and to then reconstruct a medical image representing at least a portion of the fetus. This example is described with reference to Fig. 3, which is a schematic diagram illustrating an example of a medical imaging system 200 and a monitoring system 100 for providing a fetal MCG signal 130, in accordance with some aspects of the present disclosure. It is noted that any of the operations described above as being performed by the one or more processors 120 of the monitoring system 100, may also be performed within the context of the medical imaging system 200, and that for the sake of brevity, not all examples of the monitoring system 100 are duplicated here.

In this example, the medical imaging system 200 comprises the monitoring system 100, and one or more processors 210; and
- wherein the one or more processors 210 of the medical imaging system are configured to receive the fetal MCG signal 130; and
- i) trigger the acquisition of medical image data by the medical imaging system 200, using the fetal MCG signal 130; and
- reconstruct a medical image representing at least a portion of the fetus using the acquired medical image data;
   or
- ii) record the fetal MCG signal 130 during the acquisition of medical image data by the medical imaging system 200;
- select medical image data from the acquired medical image data using the recorded fetal MCG signal 130; and
- reconstruct a medical image representing at least a portion of the fetus using the selected medical image data.

In this example, the medical imaging system 200 may be provided by various types of medical imaging system, including for example an MRI system, a (spectral) computed tomography "CT" imaging system, a (spectral) X-ray projection imaging system, a positron emission tomography "PET" imaging system, a single photon emission computed tomography "SPECT" imaging system, and so forth. In the example of the medical imaging system 200 being an MRI system, the medical image data may be referred-to as k-space data, or raw data.

By using the fetal MCG signal 130 to i) trigger the acquisition of medical image data, or to ii) select medical image data for reconstruction, a medical image representing at least a portion of the fetus may be reconstructed in which cardiac-induced motion is reduced. In the former case, i.e. i), the acquisition of medical image data may be triggered at one or more specified times in the cardiac cycle. For instance the acquisition of medical image data may be triggered at a time between the systolic and diastolic phases, or at a peak of the so-called R wave, or at another time in the cardiac cycle. In response to the trigger, data may then be acquired for a specified period. The specified period may be instantaneous, or a longer period. Reconstructing the medical image of the fetus using medical image data that is acquired from a common period in the cardiac cycle subsequent to the triggering at a common time in the cardiac cycle, results in reduced cardiac motion because the data used to reconstruct the image represents the fetal heart in the same state. This type of triggering may be referred-to as prospective gating. In the latter case, i.e. ii), the medical image data may be acquired continuously over a period of time, and the recorded fetal MCG signal 130 is used to select medical image data that corresponds to one or more specified times in the cardiac cycle. The selected data may correspond to a specified period in the cardiac cycle subsequent to the triggering. Using the selected medical image data that is acquired from a common period in the cardiac cycle subsequent to the triggering at a common time in the cardiac cycle, to reconstruct a medical image of the fetus, also results in reduced cardiac motion in the medical image because the data used to reconstruct the image likewise represents the fetal heart in the same state. This type of triggering may be referred-to as retrospective gating. Known medical image reconstruction techniques may be used to generate the reconstructed medical image.

In a related example, the one or more processors 120 of the monitoring system also generate a maternal MCG signal, the maternal MCG signal is received by the one or more processors 210 of the medical imaging system, and the maternal MCG signal is used, in a similar manner to that described above, to reconstruct a medical image representing at least a portion of the pregnant mother.

In this example, the one or more processors 210 of the medical imaging system are configured to receive the maternal MCG signal; and
- i) trigger the acquisition of medical image data by the medical imaging system 200, using the maternal MCG signal; and
- reconstruct a medical image representing at least a portion of the pregnant mother using the acquired medical image data;
   or
- ii) record the maternal MCG signal during the acquisition of medical image data by the medical imaging system 200;
- select medical image data from the acquired medical image data using the recorded maternal MCG signal; and
- reconstruct a medical image representing at least a portion of the pregnant mother using the selected medical image data.

The above operations may be performed for the maternal MCG signal using the same techniques described above for the fetal MCG signal. This example advantageously provides both a fetal image and a maternal image in which cardiac-induced motion is reduced.

In a related example, the acquisition of medical image data by the medical imaging system 200 may take place concurrently for the medical image representing at least a portion of the fetus and for the medical image representing at least a portion of the pregnant mother. Advantageously, this provides the medical image data for both a fetal image and a maternal image during the same imaging procedure.

In another example, the medical imaging system generates a timing signal corresponding to a generation of magnetic fields by the medical imaging system 200, and the timing signal is used to reduce interference to the composite MCG signals.

In this example, the medical imaging system 200 is configured to generate a timing signal corresponding to a generation of magnetic fields by the medical imaging system 200; and
- wherein the one or more processors 120 of the monitoring system are further configured to receive the timing signal; and
- i) synchronise the acquisition of the composite MCG signals, using the timing signal, for reducing interference to the composite MCG signals from the magnetic fields generated by the medical imaging system 200;
   or
- ii) compensate for interference to the composite MCG signals from the magnetic fields generated by the medical imaging system 200, using the timing signal.

In this example, the timing signal may correspond to a time of gradient field generation by an MRI system, or a time of the emission of X-ray radiation by a CT or an X-ray imaging system, for example. The magnetic fields that are generated at such times may interfere with the composite signals detected by the magnetic field sensors 110_{1..i}, and thereby degrade the quality of the fetal MCG signal 130 that is generated by the monitoring system 100. In this example, i), the acquisition of the composite MCG signals may be synchronised so as to avoid acquiring the composite MCG signals during periods when the magnetic fields are generated by the medical imaging system 200. Alternatively, ii), compensation may be provided for interference to the composite MCG signals from the magnetic fields generated by the medical imaging system 200. In the latter case, the magnetic field sensors 110_{1..i} may each comprise at least one magnetic field compensation coil, and the timing signal may be used to apply signals to the magnetic field compensation coils in order to reduce the interference to the composite MCG signals from the magnetic fields generated by the medical imaging system. Alternatively, the one or more processors of the monitoring system may calculate, using the timing signal, expected background signals corresponding to the magnetic fields generated by the medical imaging system, and subtract, actively in real-time, or retrospectively, the expected background signals from the received composite signals. The calculation of the expected background signals may be performed using a volumetric model to estimate the expected interference. The model may be provided using finite element magnetic field modelling techniques, for example.

An example of the medical imaging system 200 is illustrated in Fig. 3. It is noted that the medical imaging system 200 may include one or more of: a patient support 220, a display, such as the monitor 230 illustrated in Fig. 3, for displaying the fetal MCG signal 130, other outputs generated by the one or more processors 120, and so forth; and a user input device (not illustrated in Fig. 3) configured to receive user input in relation to the operations performed by the one or more processors 210 of the medical imaging system 200, such as a keyboard, a mouse, a touchscreen, and so forth.

In another example, a computer-implemented method of reconstructing a medical image, is provided. The method includes:
- receiving S 110, from a plurality of magnetic field sensors 110_{1..i}, composite MCG signals generated by the magnetic field sensors 110_{1..i}, the composite MCG signals comprising a maternal MCG signal representing magnetic fields generated by a beating heart of a pregnant mother and a fetal MCG signal 130 representing magnetic fields generated by a beating heart of her fetus;
- analysing S 120 the composite MCG signals to extract the fetal MCG signal 130 from the composite MCG signals; and
- outputting S 130 the fetal MCG signal 130; and
- i) triggering the acquisition of medical image data by the medical imaging system 200, using the fetal MCG signal 130; and
- reconstructing a medical image representing at least a portion of the fetus using the acquired medical image data;
   or
- ii) recording the fetal MCG signal 130 during the acquisition of medical image data by the medical imaging system 200;
- selecting medical image data from the acquired medical image data using the recorded fetal MCG signal 130; and
- reconstructing a medical image representing at least a portion of the fetus using the selected medical image data.

In another example, a computer program product, is provided. The computer program product comprises instructions which when executed by one or more processors, cause the one or more processors to carry out a method of reconstructing a medical image. The method includes:
- receiving S 110, from a plurality of magnetic field sensors 110_{1..i}, composite MCG signals generated by the magnetic field sensors 110_{1..i}, the composite MCG signals comprising a maternal MCG signal representing magnetic fields generated by a beating heart of a pregnant mother and a fetal MCG signal 130 representing magnetic fields generated by a beating heart of her fetus;
- analysing S 120 the composite MCG signals to extract the fetal MCG signal 130 from the composite MCG signals; and
- outputting S 130 the fetal MCG signal 130; and
- i) triggering the acquisition of medical image data by the medical imaging system 200, using the fetal MCG signal 130; and
- reconstructing a medical image representing at least a portion of the fetus using the acquired medical image data;
   or
- ii) recording the fetal MCG signal 130 during the acquisition of medical image data by the medical imaging system 200;
- selecting medical image data from the acquired medical image data using the recorded fetal MCG signal 130; and
- reconstructing a medical image representing at least a portion of the fetus using the selected medical image data.

The above examples are to be understood as illustrative of the present disclosure, and not restrictive. Further examples are also contemplated. For instance, the examples described in relation to the monitoring system 100, may also be provided by the corresponding computer program product, or by the computer-readable storage medium, or they may be incorporated within the medical imaging system 200, or within the corresponding computer program product, or within the corresponding computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A monitoring system (100) for providing a fetal cardiac signal, the monitoring system comprising:
a plurality of magnetic field sensors (110_{1..i}); and
one or more processors (120);
wherein the one or more processors (120) are configured to:
receive (S 110) composite magnetocardiogram, MCG, signals generated by the magnetic field sensors (110_{1..i}), the composite MCG signals comprising a maternal MCG signal representing magnetic fields generated by a beating heart of a pregnant mother and a fetal MCG signal (130) representing magnetic fields generated by a beating heart of her fetus;
analyse (S120) the composite MCG signals to extract the fetal MCG signal (130) from the composite MCG signals; and
output (S130) the fetal MCG signal (130) to provide the fetal cardiac signal.

2. The monitoring system according to claim 1, wherein the analysing (S120) the composite MCG signals, further comprises extracting the maternal MCG signal from the composite MCG signals; and
wherein the one or more processors (120) are further configured to output the maternal MCG signal to provide a maternal cardiac signal.

3. The monitoring system according to any previous claim, wherein the plurality of magnetic field sensors (110_{1..i}) comprise a first magnetic field sensor for coupling to a thorax of the pregnant mother, and a second magnetic field sensor for coupling to an abdomen of the pregnant mother.

4. The monitoring system according to any previous claim, wherein the magnetic field sensors (110_{1..i}) comprise inductive sensors, or quantum sensors such as optically pumped magnetometer, OPM, sensors, or nitrogen vacancy diamond sensors.

5. The monitoring system according to any previous claim, wherein at least one of:
the magnetic field sensors (110_{1..i}) further comprise magnetic shielding for reducing interference to the composite MCG signals from background magnetic fields;
the monitoring system further comprises a filter configured to filter the composite MCG signals for reducing interference to the composite MCG signals from background magnetic fields;
the monitoring system further comprises one or more reference magnetic field sensors configured to detect background magnetic fields, and wherein the one or more processors (120) are further configured to compensate for interference to the composite MCG signals from the background magnetic fields, using the detected background magnetic fields;
the magnetic field sensors (110_{1..i}) each comprise at least one magnetic field compensation coil, and the one or more processors (120) are further configured to apply signals to the magnetic field compensation coils for reducing interference to the composite MCG signals from background magnetic fields.

6. The monitoring system according to any previous claim, wherein the analysing (S120) the composite MCG signals comprises:
inputting the composite MCG signals into a neural network; and
generating the fetal MCG signal (130), from the composite MCG signals, in response to the inputting, using the neural network; and
wherein the neural network is trained to generate the fetal MCG signal (130), from the composite MCG signals, using training data comprising a plurality of training composite signals comprising a maternal MCG signal representing magnetic fields generated by a beating heart of a pregnant mother and a fetal MCG signal (130) representing magnetic fields generated by a beating heart of her fetus, and for each training composite signal, a corresponding ground truth fetal MCG signal or a corresponding ground truth fetal ECG signal.

7. The monitoring system according to claim 6, wherein the neural network is trained to generate the fetal MCG signal (130) from the composite MCG signals, by:
for each of a plurality of the training composite signals:
inputting the training composite signal into the neural network;
generating a fetal MCG signal (130), from the training composite signal, in response to the inputting, using the neural network; and
adjusting parameters of the neural network based on a difference between the fetal MCG signal (130) generated by the neural network, and the corresponding ground truth fetal MCG signal, or the corresponding ground truth fetal ECG signal, respectively; and
repeating the inputting, the generating, and the adjusting, until a stopping criterion is met.

8. The monitoring system according to claim 6 or claim 7, wherein at least some of the training composite signals comprise synthetically-generated training composite signals, and wherein the synthetically-generated training composite signals each represent a maternal MCG signal and a fetal MCG signal (130).

9. The monitoring system according to any one of claims 6-8, further comprising at least one vital signs sensor (140); and
wherein the one or more processors (120) are further configured to:
receive at least one vital signs signal generated by the at least one vital signs sensor, the vital signs signal representing a vital sign of the pregnant mother;
input the at least one vital signs signal into the neural network; and
generate the fetal MCG signal (130), from the composite MCG signals, based further on the at least one vital signs signal; and
wherein the training data used to train the neural network further comprises for the at least one vital signs sensor, training vital signs signals corresponding to the training composite signals.

10. The monitoring system according to any one of claims 6-9, further comprising at least one motion sensor; and
wherein the one or more processors (120) are further configured to:
receive at least one motion signal generated by the at least one motion sensor, the motion signal representing a motion of the pregnant mother;
input the motion signal into the neural network; and
generate the fetal MCG signal (130), from the composite MCG signals, based further on the at least one motion signal; and
wherein the training data used to train the neural network further comprises for the at least one motion sensor, training motion signals corresponding to the training composite signals.

11. A medical imaging system (200) comprising the monitoring system (100) according to any one of claims 1 - 10; and
one or more processors (210); and
wherein the one or more processors (210) of the medical imaging system are configured to receive the fetal MCG signal (130); and
i) trigger the acquisition of medical image data by the medical imaging system (200), using the fetal MCG signal (130); and
reconstruct a medical image representing at least a portion of the fetus using the acquired medical image data;
or
ii) record the fetal MCG signal (130) during the acquisition of medical image data by the medical imaging system (200);
select medical image data from the acquired medical image data using the recorded fetal MCG signal (130); and
reconstruct a medical image representing at least a portion of the fetus using the selected medical image data.

12. The medical imaging system according to claim 11 when dependent on claim 2, wherein the one or more processors (210) of the medical imaging system are configured to receive the maternal MCG signal; and
i) trigger the acquisition of medical image data by the medical imaging system (200), using the maternal MCG signal; and
reconstruct a medical image representing at least a portion of the pregnant mother using the acquired medical image data;
or
ii) record the maternal MCG signal during the acquisition of medical image data by the medical imaging system (200);
select medical image data from the acquired medical image data using the recorded maternal MCG signal; and
reconstruct a medical image representing at least a portion of the pregnant mother using the selected medical image data.

13. The medical imaging system according to any one of claims 11 - 12, wherein the medical imaging system is further configured to generate a timing signal corresponding to a generation of magnetic fields by the medical imaging system (200); and
wherein the one or more processors (120) of the monitoring system are further configured to receive the timing signal; and
i) synchronise the acquisition of the composite MCG signals, using the timing signal, for reducing interference to the composite MCG signals from the magnetic fields generated by the medical imaging system (200);
or
ii) compensate for interference to the composite MCG signals from the magnetic fields generated by the medical imaging system (200), using the timing signal.

14. A computer-implemented method of providing a fetal cardiac signal, the method comprising:
receiving (S 110), from a plurality of magnetic field sensors (110_{1..i}), composite magnetocardiogram, MCG, signals generated by the magnetic field sensors (110_{1..i}), the composite MCG signals comprising a maternal MCG signal representing magnetic fields generated by a beating heart of a pregnant mother and a fetal MCG signal (130) representing magnetic fields generated by a beating heart of her fetus;
analysing (S120) the composite MCG signals to extract the fetal MCG signal (130) from the composite MCG signals; and
outputting (S130) the fetal MCG signal (130) to provide the fetal cardiac signal.

15. A computer program product comprising instructions which when executed by one or more processors (120), cause the one or more processors to carry out the method according to claim 14.
